# EUROPÄISCHE PATENTSCHRIFT

(11) **EP 3 704 681 B1**
(45) Veröffentlichungstag und Bekanntmachung des Hinweises auf die Patenterteilung: **19.03.2025**
(21) Anmeldenummer: 18799481.9
(22) Anmeldetag: 02.11.2018
(51) Int. Cl.: G08B 21/22, B60N 2/00, G08B 21/24

(54) **KINDERSITZÜBERPRÜFUNGSSYSTEM UND KINDERSITZÜBERPRÜFUNGSVERFAHREN**
CHILD SAFETY SEAT CHECKING SYSTEM, AND CHILD SAFETY SEAT CHECKING METHOD
SYSTÈME DE VÉRIFICATION DE SIÈGE ENFANT ET PROCÉDÉ DE VÉRIFICATION DE SIÈGE ENFANT

(30) Priorität: 03.11.2017 DE 202017106647 U
(43) Veröffentlichungstag der Anmeldung: 09.09.2020
(73) Patentinhaber: CYBEX GmbH, 95448 Bayreuth (DE)
(72) Erfinder: KLAMT, Solveig, 95448 Bayreuth (DE)
(74) Vertreter: Meissner Bolte Partnerschaft mbB
(86) Internationale Anmeldenummer: PCT/EP2018/079993
(87) Internationale Veröffentlichungsnummer: WO 2019/086599

(56) Entgegenhaltungen:
- US-A1- 2004 160 320
- US-A1- 2011 241 867
- US-A1- 2014 184 404
- US-A1- 2016 379 466
- US-A1- 2017 282 791
- US-B1- 8 058 983

## Beschreibung

Die Erfindung betrifft ein Kindersitzüberprüfungssystem und ein Kindersitzüberprüfungsverfahren.

Aus US 2017/0021800 A1 ist ein Fahrzeug-Kindersitz bekannt, der eine Einrichtung umfasst, die einen Status eines Rückhaltesystems des Kindersitzes erfasst und ausgibt. Weiterhin wird eine Steuerungseinrichtung offenbart, die an den OBD-II-Port des Kraftfahrzeugs angeschlossen werden kann und den Status einer Fahrzeug-Zündung detektiert. Wenn die Zündung ausgeschaltet ist und das Rückhaltesystem einen Status angibt, der darauf schließen lässt, dass sich ein Kind in dem Kindersitz befindet, wird nach einer vorbestimmten Zeitspanne ein Alarmsignal generiert. Die Zuverlässigkeit und Aussagekraft von gemäß diesem Stand der Technik generierten Alarmsignalen wird als verbesserungswürdig angesehen.

Aus US 2014/184404 A1 sind Systeme und Verfahren bekannt, die für einen Alarm oder eine Benachrichtigung sorgen, dass sich ein Kind in einem Autositz befindet. Die Systeme bestimmen im Allgemeinen, ob sich ein Kind auf dem Sitz befindet und dass sich der Sitz und damit das Fahrzeug nicht bewegt. Beim Erkennen beider Situationen wird die Benachrichtigung oder der Alarm aktiviert. Das System sorgt im Allgemeinen für einen Alarm an ein Smartphone oder ein anderes Fernbenachrichtigungsgerät, um die Wahrscheinlichkeit zu erhöhen, dass der Alarm empfangen wird. Die Zuverlässigkeit und Aussagekraft von gemäß diesem Stand der Technik generierten Alarmsignalen wird ebenfalls als verbesserungswürdig angesehen.

Es ist daher Aufgabe der Erfindung, ein Kindersitzüberprüfungssystem sowie ein entsprechendes Kindersitzüberprüfungsverfahren vorzuschlagen, wobei auf zuverlässige Art und Weise das Risiko reduziert werden soll, dass ein Kind ungewollt in dem Fahrzeug zurückbleibt, wobei insbesondere falsche Alarme vermieden werden sollen oder die Wahrscheinlichkeit dafür zumindest reduziert werden soll. Weiterhin soll insbesondere die Variabilität der Überprüfung verbessert werden. Vorzugsweise soll es möglich sein, dass das Kind in dem Fahrzeug - zumindest unter bestimmten Umständen - bewusst zurückgelassen wird, ohne dass dies mit größeren Beeinträchtigungen für den Benutzer des Überprüfungssystems verbunden ist.

Diese Aufgabe wird hinsichtlich eines Kindersitzüberprüfungssystems durch die Merkmale des Anspruchs 1 gelöst. Weiterhin wird die Aufgabe durch die Merkmale des Anspruchs 14 betreffend ein Kindersitzüberprüfungsverfahren gelöst.

Insbesondere wird die Aufgabe gelöst durch ein Kindersitzüberprüfungssystem, umfassend zumindest einen Kindersitz sowie eine erste Überprüfungseinrichtung zum Überprüfen, ob sich ein Kind in dem Kindersitz befindet, und eine zweite Überprüfungseinrichtung zum Überprüfen, ob eine das Kind betreuende Person anwesend ist.

Unter einer Überprüfungseinrichtung ist insbesondere eine Einrichtung zu verstehen, die auf Grundlage einer (beispielsweise eigenen) Messung oder eines sonstigen Inputs (beispielsweise aufgrund einer Messung einer weiteren Einrichtung, insbesondere Sensoreinrichtung) eine Feststellung darüber erlaubt, ob ein gewisser Zustand oder Status (beispielsweise "Kind im Kindersitz" oder "betreuende Person anwesend" oder "Wachheitsstatus des Kindes") vorliegt oder nicht. Dies gilt sowohl für die hier genannte erste und zweite Überprüfungseinrichtung als auch für nachfolgende (insbesondere die nachfolgende dritte und vierte) Überprüfungseinrichtungen. Vorzugsweise umfassen die jeweiligen Überprüfungseinrichtungen jedoch Sensoreinrichtungen, die durch eine Messung (insbesondere eines entsprechenden Parameters) feststellen, ob ein Zustand bzw. Status vorliegt oder nicht. Unter einer Überprüfungseinrichtung ist insbesondere auch zu verstehen, dass eine Einrichtung (z. B. App) auf einem (mobilen) Gerät vorliegt, die auf Grundlage eines entsprechenden Inputs (beispielsweise Messung einer gegenüber dem Gerät externen Sensoreinrichtung) überprüfen kann, ob sich ein Kind in dem Kindersitz befindet oder nicht (im Beispiel der ersten Überprüfungseinrichtung). Die (jeweilige) Überprüfungseinrichtung kann durch nur eine Baugruppe (z.B. definiert durch ein Gehäuse) ausgebildet sein oder sich auf mehrere Baugruppen (z. B. definiert durch mehrere Gehäuse) verteilen. Einzelne Komponenten, wie beispielsweise Sensoren, können mehreren Überprüfungseinrichtung zugeordnet sein.

Gemäß einem ersten bevorzugten Aspekt der Erfindung ist die zweite Überprüfungseinrichtung in einem Betriebszustand des Kindersitzüberprüfungssystems (zumindest teilweise) tragbar ausgebildet. Insbesondere kann die zweite Überprüfungseinrichtung zumindest teilweise durch ein mobiles Gerät (z. B. Smartphone) ausgebildet werden oder Bestandteil eines solchen mobilen Gerätes sein. Unter einer tragbaren Einrichtung ist eine Einrichtung zu verstehen, die (z. B. als Smartphone oder Bestandteil eines Smartphones) durch die das Kind betreuende Person getragen werden kann. Verschiedene Typen derartiger mobiler Geräte werden weiter unten noch im Einzelnen genannt. Durch eine derartige Überprüfungseinrichtung kann die Anwesenheit der das Kind betreuenden Person auf einfache Art und Weise unmittelbar mit der Person verknüpft werden (beispielsweise wenn die Person ihr Smartphone bei sich trägt). Dadurch ist die Überprüfung vergleichsweise zuverlässig. Unter einem Betriebszustand des Kindersitzüberprüfungssystems ist ein Zustand desselben zu verstehen, in dem ein Überprüfen, ob sich ein Kind in dem Kindersitz befindet bzw. ob eine das Kind betreuende Person anwesend ist, erfolgen kann. Insbesondere kann diese Überprüfung also erfolgen, wenn die zweite Überprüfungseinrichtung getragen wird (insbesondere nicht fest, mit dem Fahrzeug verbunden ist). Durch eine zumindest teilweise mobile zweite Überprüfungseinrichtung ist es nicht notwendig, wie beispielsweise in US 2017/0021800 A1, beschrieben, die zweite Überprüfungseinrichtung an die Fahrzeugelektronik via OBD-II-Port anzuschließen. Dieser Port kann mitunter vergleichsweise schwer zugänglich sein.

Alternativ oder zusätzlich ist die zweite Überprüfungseinrichtung gemäß einem zweiten bevorzugten Aspekt der Erfindung unabhängig von einem An-/AusZustand des Fahrzeuges arbeitend ausgebildet. Unter einem Ein-/Aus-Zustand ist insbesondere ein Zustand zu verstehen, bei dem die Zündung entweder an ist (Ein-Zustand) oder aus ist (Zündung aus). Insbesondere gemäß dem zweiten Aspekt kann ein zuverlässiger Alarm generiert werden, wobei die Wahrscheinlichkeit für einen Fehlalarm reduziert ist. Insbesondere wurde erkannt, dass eine Kopplung mit einem Ein-/Aus-Zustand (Zündungszustand) oftmals zu einem Fehlalarm führt, da es in manchen Situationen von den Passagieren des Fahrzeugs gewollt ist, in den Fahrzeugsitzen zu bleiben (während das Kind in dem Kindersitz angeschnallt bleibt), während die Zündung aus ist.

Im Allgemeinen ist unter einer Anwesenheit einer das Kind betreuenden Person vorzugsweise zu verstehen, dass sich die betreuende Person in oder (unmittelbar) an dem Fahrzeug befindet oder ggf. näher als in einem vorbestimmten Maximalabstand vom Kindersitz und/oder Fahrzeug befindet. Der Maximalabstand ist vorzugsweise kleiner als 50 m, insbesondere kleiner als 25 m.

Gemäß der Erfindung ist eine dritte Überprüfungseinrichtung vorgesehen, wobei die dritte Überprüfungseinrichtung zur Überprüfung des Wachheits-Status des Kindes ausgebildet ist. Unter einem Wachheits-Status des Kindes ist ein Status zu verstehen, der (unmittelbar) das (anwesende) Kind betrifft, nämlich die Frage, ob das Kind wach ist oder schläft. Nicht von der Erfindung umfasst ist beispielsweise, ob das Kind eine erhöhte Körpertemperatur hat oder eine normale. Der Status des Kindes betrifft also (unmittelbar) die physische Konstitution des Kindes. Durch eine derartige Überprüfungseinrichtung kann insbesondere ein Bedienungskomfort bei der Verwendung des Kindersitzes verbessert werden. Beispielsweise ist es möglich, dass aufgrund einer Feststellung, dass das Kind schläft, die bewusste Entscheidung getroffen wird, das Fahrzeug zu verlassen (zumindest für eine vorübergehende Zeit), wobei dann die Möglichkeit besteht, durch die Überwachung des Schlafes des Kindes sofort zum Fahrzeug zurückzukehren, wenn das Kind aufwacht.

Gemäß einem vierten bevorzugten Aspekt der Erfindung (der insbesondere mit dem ersten und/oder zweiten und/oder dritten Aspekt kombiniert werden kann) wird eine vierte Überprüfungseinrichtung vorgeschlagen zur Überprüfung einer Kindersitzumgebung, insbesondere einer Kindersitzumgebungstemperatur. Eine Überprüfung einer Kindersitzumgebung soll insbesondere eine Überprüfung einer an den Kindersitz anstehenden Luft bzw. eine Überprüfung des Raumvolumens, in dem der Kindersitz aufgenommen ist, sein. Besonders bevorzugt wird hierbei eine Temperatur der Umgebungsluft überprüft. Gegebenenfalls wäre es auch denkbar, dass alternativ oder zusätzlich weitere Parameter, wie beispielsweise eine Luftfeuchte und/oder ein Sauerstoffanteil und/oder ggf. schädliche Gase (wie z. B. Kohlenmonoxid) überprüft werden.

Erste, zweite, dritte Überprüfungseinrichtung sind nicht nur konfiguriert, die entsprechende Überprüfung durchzuführen, sondern auch ein Ergebnis der Überprüfung auszugeben bzw. weiterzugeben, nämlich an eine entsprechende Steuereinrichtung. Insbesondere umfassen erste, zweite, dritte Überprüfungseinrichtung eine (jeweilige) Ausgabeeinrichtung zur Ausgabe eines Ergebnisses der Überprüfung, insbesondere an eine oder mehrere Steuereinrichtungen.

Wenn eine physikalische Größe zu bestimmen ist (z. B. Kraft, Gewicht, Temperatur und/oder Luftfeuchte) sind entsprechende Messeinrichtungen vorzugsweise derart ausgebildet, dass mindestens vier, vorzugswiese mindestens zehn voneinander unterscheidbare Messwerte messbar sind. Eine (jeweilige) ggf. vorgesehene Messeinrichtung zur Messung der Temperatur soll vorzugswiese eine Temperaturmessung erlauben, die maximal 2 °C, vorzugsweise maximal 0,5 °C von der tatsächlichen Temperatur abweicht und/oder die einen Bereich von mindestens 5 °C, ggf. mindestens 20 °C abdeckt.

Die dritte und ggfs. vierte Überprüfungseinrichtung sind (funktionell und/oder strukturell) zusätzlich zu der ersten Überprüfungseinrichtung vorgesehen. Eine Überprüfung durch die dritte und ggfs. vierte Überprüfungseinrichtung erfolgt separat (zeitlich davor, zeitlich überlappend oder nach) einer Überprüfung der Anwesenheit des Kindes. Beispielsweise wird also eine Information, ob sich überhaupt ein Kind im Kindersitz befindet und eine Information betreffend beispielsweise eine Kindersitzumgebungstemperatur separat bereitgestellt. Dadurch kann die Sicherheit verbessert werden und ggf. ein Fehlalarm vermieden werden. Insbesondere ist die dritte und/oder vierte Überprüfungseinrichtung nicht vorgesehen (nur) die Anwesenheit des Kindes zu detektieren, sondern vorzugsweise weitere Informationen betreffend das Kind zu gewinnen.

Erfindungsgemäß ist mindestens eine, insbesondere die eben erwähnte, Steuereinrichtung vorgesehen, die konfiguriert ist, in Abhängigkeit einer Überprüfung durch die erste, zweite, dritte und ggfs. vierte Überprüfungseinrichtung, mindestens ein Ergebnis aus einem Vergleich der Überprüfungen auszugeben, nämlich an eine, vorzugsweise akustische und/oder optische, Anzeigeeinrichtung. Die Steuereinrichtung kann ggf. mit der ersten, zweiten, dritten und/oder vierten, insbesondere mit der ersten, dritten und vierten (z. B. in/an dem Kindersitz) oder mit der zweiten (z. B. in/an einem mobilen Gerät, insbesondere Smartphone) Überprüfungseinrichtung, in einer gemeinsamen Baugruppe bzw. einem gemeinsamen Gehäuse angeordnet sein, wobei das Gehäuse vorzugsweise durch ein mobiles Gerät (z. B. Smartphone) gebildet wird. Auch die Anzeigeeinrichtung ist ggf. innerhalb desselben bzw. auf demselben Gehäuse vorgesehen wie auch die Steuereinrichtung und/oder die erste, zweite, dritte und/oder vierte, insbesondere die zweite, Überprüfungseinrichtung. Die (jeweilige) Steuereinrichtung umfasst vorzugsweise mindestens einen (Micro-)Prozessor und/oder (Micro-)Controller.

Der Kindersitz kann eine elektrische Leistungsversorgung (z. B. Batterie) aufweisen und/oder mindestens einen Anschluss für eine elektrische Leistungsversorgung und/oder mindestens einen Anschluss zur (drahtlosen und/oder drahtgebundenen) Signalübertragung. Vorzugsweise ist die Anzeigeeinrichtung konfiguriert, sämtliche

Ergebnisse aus den Überprüfungen der Überprüfungseinrichtungen anzuzeigen und/oder ein Alarmsignal auszugeben, falls bzw. sobald ein unerwünschter Zustand im Hinblick auf das Kind und/oder die Anwesenheit der betreuenden Person festgestellt wird (z. B.: Kind im Kindersitz, betreuende Person nicht anwesend), insbesondere durch einen Vergleich der Überprüfungsergebnisse, vorzugsweise unter Berücksichtigung sämtlicher Überprüfungsergebnisse der (vier) Überprüfungseinrichtungen.

Vorzugsweise bildet mindestens eine, insbesondere mindestens eine obige, Anzeigeeinrichtung eine Baueinheit (z. B. in Form eines Smartphones) mit der zweiten Überprüfungseinrichtung oder zumindest mit Teilen davon.

Alternativ oder zusätzlich kann mindestens eine, insbesondere mindestens eine obige, Anzeigeeinrichtung eine Baueinheit mit dem Kindersitz ausbilden.

Unter einer akustischen Anzeige (Ausgabe) ist insbesondere ein Warnton/Warnsignal zu verstehen und/oder eine akustische Nachricht (Sprachnachricht). Unter einer optischen Anzeige ist insbesondere das Aufleuchten einer Lichtquelle (z. B. LED) und/oder eine Anzeige auf einem Display und/oder eine optische Nachricht (Textnachricht) zu verstehen. Alternativ zu einer akustischen und/oder optischen Anzeigeeinrichtung kann auch eine Anzeige (Ausgabe) bzw. ein Alarm durch Vibration oder auf noch andere Art und Weise erfolgen.

Erste, dritte und/oder vierte Überprüfungseinrichtung (bzw. eine entsprechende erste, dritte und/oder vierte Sensoreinrichtung) können zumindest teilweise an dem und/oder in dem Kindersitz angeordnet sein, wobei die vierte Überprüfungseinrichtung (Sensoreinrichtung) vorzugsweise zumindest teilweise an einer Außenseite des Kindersitzes angeordnet ist.

Die zweite Überprüfungseinrichtung ist vorzugsweise (zumindest überwiegend) außerhalb des Kindersitzes angeordnet. Beispielsweise kann lediglich eine Sende- und/oder Empfangseinrichtung auch im Fall der zweiten Überprüfungseinrichtung an dem und/oder in dem Kindersitz angeordnet sein, beispielsweise wenn ein mobiles Gerät (z. B. Smartphone) seinen Abstand zu dem Kindersitz bestimmt. Alternativ kann eine derartige Abstandsbestimmung jedoch auch durch eine entsprechende Einrichtung in und/oder an dem Kindersitz erfolgen. Insbesondere bei der zweiten Überprüfungseinrichtung kann vorgesehen sein, dass sich diese ausführungsgemäß über mehrere Orte (z. B. Kindersitz und mobiles Gerät) verteilt und letztlich Sender/Empfänger umfasst, die durch gemeinsame Kommunikation eine Feststellung ihres Abstandes zueinander erlauben (z. B. durch eine Signalabschwächung aufgrund einer vergrößerten Entfernung).

Die erste Prüfungseinrichtung umfasst vorzugsweise mindestens einen Rückhalteeinrichtungssensor, der konfiguriert ist, einen Status mindestens einer Rückhalteeinrichtung, wie beispielsweise eines Haltegurtes und/oder eines Fangkörpers, festzustellen.

Weiterhin kann die erste (und/oder dritte) Überprüfungseinrichtung mindestens einen Gewichtssensor (vorzugsweise in/an dem Kindersitz, bei ggf. mehrteiligen Kindersitzen also beispielsweise an einem Sitzelement und/oder der Basis) umfassen, der auf ein in dem Kindersitz befindliches Kind bzw. dessen Gewichtskraft entsprechend reagiert. Ein entsprechender Gewichtssensor kann auf bzw. an einem Sitzabschnitt und/oder einen Rückenlehnenabschnitt des Kindersitzes angeordnet sein.

In Ausführungsformen kann die erste (und/oder dritte) Überprüfungseinrichtung mindestens eine optische Einrichtung, wie z. B. eine (Video-)Kamera, umfassen die insbesondere feststellt, ob ein Kind in dem Kindersitz sitzt oder ob sich das Kind bewegt. Die (Video-)Kamera kann insbesondere in bzw. an einem Tragegriff (Tragebügel) des Kindersitzes angeordnet sein.

In einer Ausführungsform kann die Rückhalteeinrichtung ein Gurtsystem umfassen. In diesem Fall kann die erste Überprüfungseinrichtung eine Sensoreinrichtung umfassen, die feststellt, ob eine oder mehrere (ggf. alle) Rastzungen mit einem entsprechenden Gurtschloss verbunden sind. In einem verbundenen (arretierten) Zustand kann ggf. ein elektrischer Stromkreis geschlossen werden (wenn alle Gurtzungen mit dem entsprechenden Schloss verbunden sind). Weiterhin kann ein entsprechender elektrischer Schaltkreis ggf. geöffnet sein, wenn zumindest eine Gurtzunge nicht mit einem entsprechenden Schloss verbunden ist. In einer derartigen Überprüfungseinrichtung bzw. Sensoreinrichtung kann der (ohmsche) Widerstand ein geeigneter Parameter zur Überprüfung sein. Im Allgemeinen ist es auch möglich, andere physikalische (elektromagnetische) Parameter zu detektieren, wie beispielsweise eine Kapazität oder dergleichen. Die Überprüfung des Widerstandes ist jedoch bevorzugt.

Weiterhin für den Fall, dass die Rückhalteeinrichtung ein Gurtsystem umfasst, kann die erste (und/oder dritte) Überprüfungseinrichtung einen Sensor umfassen, um eine (mechanische) Einwirkung, vorzugsweise (Zug-)Spannung, Neigung, Biegung und/oder Verwindung, auf eines oder mehrere Elemente des Gurtsystems festzustellen (beispielsweise auf einen Schultergurt, einen Beckengurt, eine Schlossaufnahme, einen Brustgurt, einen Brust-Clip etc.). Diesbezüglich wird insbesondere hinsichtlich der weiteren Ausgestaltung auf WO 2017/042286 A1 verwiesen. Alternativ oder zusätzlich kann mindestens ein Hall-Sensor zum Einsatz kommen. Alternativ oder zusätzlich können weitere elektrische Eigenschaften, wie ein Widerstand, eine Kapazität und/oder Induktivität gemessen werden (in diesem Zusammenhang wird explizit auf die Offenbarung gemäß WO 2016/128478 A1 verwiesen). Die erste Überprüfungseinrichtung kann auch einen Drucksensor umfassen (alternativ oder zusätzlich), der den Druck an einer geeigneten Stelle des Gurtsystems feststellt (was wiederum als indirekte Messung einer Gurtspannung angesehen werden kann).

Für den Fall, dass die Rückhalteeinrichtung eine (starre) Einrichtung wie beispielsweise einen Fangkörper aufweist, kann die erste (und/oder dritte) Überprüfungseinrichtung einen Drucksensor umfassen, der vorzugsweise auf einer Seite (Fläche) der (starren) Rückhalteeinrichtung aufliegt, die vorgesehen ist, um in Kontakt mit dem (im Kindersitz sitzenden) Kind zu kommen.

Die zweite Überprüfungseinrichtung kann mindestens einen fahrzeugeigenen Sensor, wie beispielsweise einen Gewichtssensor und/oder einen Zündsensor, umfassen. Bei dem Gewichtssensor kann es sich insbesondere um einen Sensor handeln, der an oder in einem im Fahrzeug integrierten Sitz (vorzugsweise Fahrersitz) angeordnet ist. Alternativ oder zusätzlich kann als fahrzeugeigener Sensor beispielsweise eine (Video-) Kamera zum Einsatz kommen. Weitere Alternativen sind möglich. In jedem Fall ist vorzugsweise bei der Verwendung eines fahrzeugeigenen Sensors eine Einrichtung vorgesehen, die eine Verbindung mit der Fahrzeugelektronik, beispielsweise über einen OBD-II-Port erlaubt (vgl. diesbezüglich auch US 2017/0021800 A1.

Alternativ oder zusätzlich kann die zweite Überprüfungseinrichtung (zumindest teilweise) ein elektronisches (End-)Gerät zur Verwendung durch die betreuende Person (z. B. Smartphone, Smartwatch, separate Überprüfungseinrichtung oder dergleichen) aufweisen. In diesem Fall kann vorzugsweise eine Entfernung zwischen dem Kindersitz und dem elektronischen (End-) Gerät (als direktes oder indirektes) Signal für die Anwesenheit der betreuenden Person herangezogen werden.

In Ausführungsformen kann die zweite Überprüfungseinrichtung ein mobiles (End-)Gerät, wie beispielsweise ein Mobiltelefon, insbesondere Smartphone, und/oder eine Uhr, insbesondere Smartwatch, und/oder einen Activity-Tracker und/oder eine Seh- und/oder eine Hörhilfe und/oder einen, insbesondere elektrischen bzw. elektronischen, Zündschlüssel und/oder ein sonstiges mobiles Gerät, insbesondere umfassend einen Transmitter und/oder Receiver und/oder Transponder, aufweisen. Ein jegliches mobiles Gerät kann mit einer (ggf. auch mechanischen) Schlüsselverbindungseinrichtung ausgestattet sein, um eine (ggf. mechanische) Verbindung mit dem (ggf. elektrischen oder elektronischen) Fahrzeugschlüssel zu ermöglichen. Unter einer Seh- und/oder eine Hörhilfe können auch Vorrichtungen verstanden werden, die nicht zwingend (aber ggf.) ein "Sehen" bzw. "Hören" verbessern, beispielsweise einen Seh- und/oder Hörfehler ausgleichen, sondern auch Vorrichtungen, die zusätzliche Informationen (z.B. eine "augmented reality" bzw. erweiterte Realität, worunter insbesondere eine computergestützte Erweiterung der Realitätswahrnehmung zu verstehen ist, z. B. "Google Glass^{™}") dem jeweiligen Sinnesorgan zuführen.Alternativ oder zusätzlich kann als zweite Überprüfungseinrichtung (oder Bestandteil einer solchen) eine Abstandsbestimmungseinrichtung vorgesehen sein, die ggf. außerhalb eines/des mobilen Gerätes angeordnet ist, z. B. am/im Kindersitz, und die konfiguriert ist, einen Abstand zwischen dem Kindersitz und einem (dem) mobilen Gerät, wie beispielsweise einem Mobiltelefon, insbesondere Smartphone, und/oder einer Uhr, insbesondere Smartwatch, und/oder einem Activity-Tracker und/oder einer Seh- und/oder Hörhilfe und/oder einem, insbesondere elektrischen bzw. elektronischen, Zündschlüssel, und/oder einem sonstigen mobilen Gerät, insbesondere umfassend einen Transmitter und/oder Receiver und/oder Transponder, zu bestimmen.

Ein mobiles Gerät kann Einrichtungen zum akustischen und/oder optischen (visuellen) und/oder haptischen (insbesondere per Vibration) Signalisieren eines Status des Kindersitzes aufweisen, insbesondere entsprechende Mittel, um eine ungewünschte Konstellation hinsichtlich des Status des Kindes per Alarm mitzuteilen. Weiterhin kann es ein derartiges mobiles (elektronisches) Gerät dem Benutzer erlauben, den Alarm, beispielsweise für eine vorbestimmte Zeitdauer, zu unterdrücken. Derartige Funktionalitäten können, wenn es sich bei dem mobilen Gerät um einen Smartdevice (z. B. Smartphone, Smartwatch oder dergleichen) handelt, durch eine entsprechende App implementiert sein.

Die dritte Überprüfungseinrichtung umfasst vorzugsweise mindestens einen Bewegungssensor und/oder eine Kamera und/oder einen Geräuschsensor und/oder mindestens einen Gewichtssensor und/oder mindestens einen Sensor zur Bestimmung eines physischen Zustandes des Kindes, wie beispielsweise einen Pulssensor und/oder einen Atmungssensor. Wenn die dritte Überprüfungseinrichtung einen Bewegungssensor umfasst, kann dazu eine/die (auch) der ersten Überprüfungseinrichtung zugeordnete Sensoreinrichtung zum Einsatz kommen, wie weiter oben erläutert. Die dritte Überprüfungseinrichtung kann auch einen Gewichtssensor umfassen, wie im Zusammenhang mit der ersten Überprüfungseinrichtung erläutert. Im allgemeinen ist ein der dritten Überprüfungseinrichtung zugeordneter Sensor vorzugsweise konfiguriert, so exakt zu messen, dass zwischen einem ruhigen (insbesondere schlafenden) Kind, das zumindest im Wesentlichen bewegungslos ist bzw. sich regelmäßig bewegt, einerseits, und einem wachen, sich insbesondere aperiodisch bewegenden Kind, oder gar einem erschrockenen bzw. wütenden Kind, das sich insbesondere ruckartig und unregelmäßig bewegt, andererseits, unterschieden werden kann. Auch kann ein und dieselbe Videokamera zum Einsatz kommen, um sowohl die Anwesenheit des Kindes im Rahmen der ersten Überprüfungseinrichtung als auch dessen Aktivität (also die Frage, ob es schläft oder wach ist) im Falle der dritten Überprüfungseinrichtung auszubilden. Die vierte Überprüfungseinrichtung kann mindestens ein Thermometer aufweisen, das es ermöglicht die Temperatur in dem Fahrzeug, in der Nähe des Kindes in dem Kindersitz, festzustellen. Alternativ oder zusätzlich kann die vierte Überprüfungseinrichtung einen Feuchtigkeitssensor und/oder einen Gassensor (insbesondere zur Feststellung von schädlichen Gasen, wie Kohlenmonoxid) aufweisen.

Erste, zweite, dritte und/oder vierte Überprüfungseinrichtung können (jeweils für sich) mehrere Sensoren und/oder einen Sensor-Array umfassen.

Vorzugsweise sind der Kindersitz und die zweite Überprüfungseinrichtung zur drahtlosen Kommunikation über eine erste drahtlose Kommunikationsverbindung, z. B. über Bluetooth, ausgebildet. Eine Messung, z. B. Abstandsmessung, auf Grundlage der ersten Kommunikationsverbindung wird vorzugsweise über die erste Kommunikationsverbindung oder eine zweite drahtlose Kommunikationsverbindung, z. B. eine Mobilfunknetzverbindung, kommuniziert. Alternativ oder zusätzlich kann bei einer Unterbrechung der ersten drahtlosen Kommunikationsverbindung eine entsprechende Information, insbesondere ein entsprechender Alarm, generiert bzw. ausgegeben werden, vorzugsweise automatisch (analog einem Babyphone) oder durch Kommunizieren mittels der zweiten drahtlosen Kommunikationsverbindung. Vorzugsweise hat die zweite drahtlose Kommunikationsverbindung eine (insbesondere um mindestens den Faktor zwei, vorzugsweise mindestens den Faktor 10) größere Reichweite als die erste drahtlose Kommunikationsverbindung. Die erste drahtlose Kommunikationsverbindung hat ggf. eine Reichweite von weniger als 100 m oder weniger als 50 m. Die zweite drahtlose Kommunikationsverbindung hat ggf. eine Reichweite von mehr als 100 m oder mehr als 10 km oder eine (im Wesentlichen) globale Reichweite.

Mehrere der ersten, zweiten, dritten und vierten Überprüfungseinrichtung können eine gemeinsame Sensoreinrichtung (wie beispielsweise einen gemeinsamen Gewichtssensor, einen gemeinsamen Sensor einer Rückhalteeinrichtung und/oder eine gemeinsame Kamera) aufweisen. Beispiele hierfür wurden bereits weiter oben erläutert.

Erste, zweite, dritte und/oder vierte Überprüfungseinrichtung und/oder Steuereinrichtung und/oder Anzeigeeinrichtung können zur drahtlosen Kommunikation (miteinander), insbesondere über Funk und/oder Bluetooth und/oder Infrarot, und/oder zur internetbasierten Kommunikation ausgebildet sein. Dadurch wird die Überprüfung weiter vereinfacht.

Vorzugsweise wird die Anwesenheit der betreuenden Person dadurch festgestellt bzw. geschätzt, dass eine Kommunikation zwischen dem Kindersitz oder Fahrzeug, in dem sich der Kindersitz befindet, und einem mobilen Gerät (z. B. Smartphone) ausgewertet wird.

Jegliches Messergebnis von zum Einsatz kommenden Sensoreinrichtungen kann an eine (die obige) oder mehrere Steuereinrichtung(en) ausgegeben werden und (insbesondere dort) mit entsprechenden Referenzwerten verglichen werden. Abhängig von einem Ergebnis eines solchen Vergleichs kann/können die Steuereinrichtung(en) feststellen ob:
- sich ein Kind im Kindersitz befindet oder nicht,
- eine betreuende Person anwesend ist (oder entfernt ist),
- das Kind schläft oder wach ist und/oder
- die äußere Umgebung (insbesondere Temperatur) in dem Fahrzeug angemessen für das Kind ist oder nicht.

Die oben genannte Aufgabe wird weiterhin gelöst durch ein Kindersitzüberprüfungsverfahren, vorzugsweise unter Verwendung eines Kindersitzüberprüfungssystems, wie oben beschrieben, umfassend:
a) Überprüfen, ob sich ein Kind in einem Kindersitz befindet;
b) Überprüfen, ob eine das Kind betreuende Person anwesend ist; und
c) Überprüfen eines Wachheitsstatus des Kindes.

Gemäß einem ersten bevorzugten Aspekt des Kindersitzüberprüfungsverfahrens erfolgt Schritt a) mittels einer Überprüfungseinrichtung, die (auch während des Überprüfens) tragbar ausgebildet ist (also insbesondere während des Überprüfens nicht zwingend an das Fahrzeug, insbesondere einen OBD-II-Port angeschlossen sein muss).

Gemäß einem zweiten bevorzugten Aspekt des Kindersitzüberprüfungsverfahrens (alternativ oder zusätzlich zum ersten bevorzugten Aspekt) wird vorgeschlagen, dass eine Überprüfungseinrichtung in Schritt b) zum Einsatz kommt, die unabhängig vom Betriebszustand des Fahrzeuges arbeitend ausgebildet ist bzw. unabhängig vom Betriebszustand des Fahrzeuges (während des Überprüfens) arbeitet.

Gemäß dem Kindersitzüberprüfungsverfahrens wird das Überprüfen eines Status, nämlich Wachheits-Status, des Kindes vorgeschlagen.

Gemäß einem vierten bevorzugten Aspekt des Kindersitzüberprüfungsverfahrens (der insbesondere mit dem ersten, zweiten und/oder dritten bevorzugten Aspekt des Kindersitzüberprüfungsverfahrens kombiniert werden kann) wird vorgeschlagen, eine Kindersitzumgebung, insbesondere eine Kindersitzumgebungstemperatur, zu überprüfen.

Vorzugsweise erfolgt eine optische und/oder akustische Anzeige, insbesondere ein optischer und/oder akustischer Alarm, insbesondere ohne dass weitere Bedingungen vorliegen müssen, vorzugsweise sofort, wenn (wobei KiKS = Kind in Kindersitz, Ba = Betreuer anwesend, Kw = Kind wach, Temperatur in akzeptablen Bereich = TiaB):
KiKS = ja, (Ba = beliebig,) (Kw = beliebig,) TiaB = nein; und/oder
KiKS = ja, Ba = nein, ggf. Kw = ja, (TiaB = beliebig).

Alternativ oder zusätzlich kann eine optische und/oder ein akustische Anzeige, insbesondere ein optischer und/oder akustischer Alarm, nicht erfolgen oder (erst) erfolgen, wenn vorzugsweise eine vorbestimmte Zeit verstrichen ist, und wenn:
KiKS = ja, Ba = nein, ggf. Kw = nein, (TiaB = beliebig).

Eine (optische und/oder akustische) Anzeige, insbesondere ein (optischer und/oder akustischer) Alarm, erfolgt vorzugsweise nicht (insbesondere auch nicht nach Verstreichen einer gewissen Zeit), wenn:
KiKS = nein, (Ba = beliebig,) (Kw = beliebig,) (TiaB = beliebig); und/oder
KiKS = ja, Ba = ja, (Kw = beliebig,) ggf. TiaB = ja; und/oder
die Anzeige bzw. der Alarm von der betreuenden Person unterdrückt wird, was vorzugsweise zumindest für eine Zeitspanne t1 möglich ist, insbesondere wenn Ba = nein und/oder Kw = ja bzw. wenn das Kind aufwacht und/oder was vorzugsweise nicht oder nur für eine vorübergehende Zeitspanne t2, vorzugsweise kleiner t1, möglich ist, wenn die Temperatur nicht in einem akzeptablen Bereich liegt.

In einer bevorzugten Ausführungsform des Kindersitzüberprüfungsverfahrens erfolgt ein Statuswechsel von Kind=wach zu Kind=schlafend nur, wenn die betreuende Person anwesend ist. Selbst wenn also das Kind einschläft, wird weiterhin der Status Kind=wach beibehalten (das Kind gilt also als wach), wenn die betreuende Person nicht anwesend ist.

Weiterhin wird ein computerlesbares Speichermedium, insbesondere ein mobiles Gerät (z. B. Smartphones), offenbart, welches Instruktionen enthält, die mindestens einen Prozessor dazu veranlassen, ein Kindersitzüberprüfungsverfahren der obigen Art durchzuführen, wenn die Instruktionen durch mindestens einen Prozessor ausgeführt werden.

Weiterhin wird ein mobiles Gerät offenbart, konfiguriert zur Durchführung des obigen Kindersitzüberprüfungsverfahrens und/oder umfassend (zumindest teilweise) ein Kindersitzüberprüfungssystem der obigen Art.

Bei dem Kindersitz kann es sich um eine Babyschale oder einen (klassischen) Kindersitz für ältere Kinder handeln. Auf jeden Fall ist der Kindersitz vorzugsweise entfernbar von dem Fahrzeug, in/auf dem er montiert werden soll, ausgebildet. Der Kindersitz kann eine Basis aufweisen (also ein Sitzelement sowie eine Basis umfassen).

Besonders bevorzugt handelt es sich bei dem Kindersitz um einen Kindersitz für einen Kraftwagen (Personenkraftwagen und/oder Lastkraftwagen).

Weitere Merkmale ergeben sich aus den Unteransprüchen.

Nachfolgend wird die Erfindung anhand von Ausführungsbeispielen näher erläutert. Hierbei zeigen:
- Fig. 1: eine Schrägansicht eines erfindungsgemäßen Kindersitzüberprüfungssystems (teilweise in Schrägansicht, teilweise schematisch);
- Fig. 2: ein schematisches erfindungsgemäßes Kindersitzüberprüfungssystem mit weiteren Details.

In der nachfolgenden Beschreibung werden für gleich und gleichwirkende Teile dieselben Bezugsziffern verwendet.

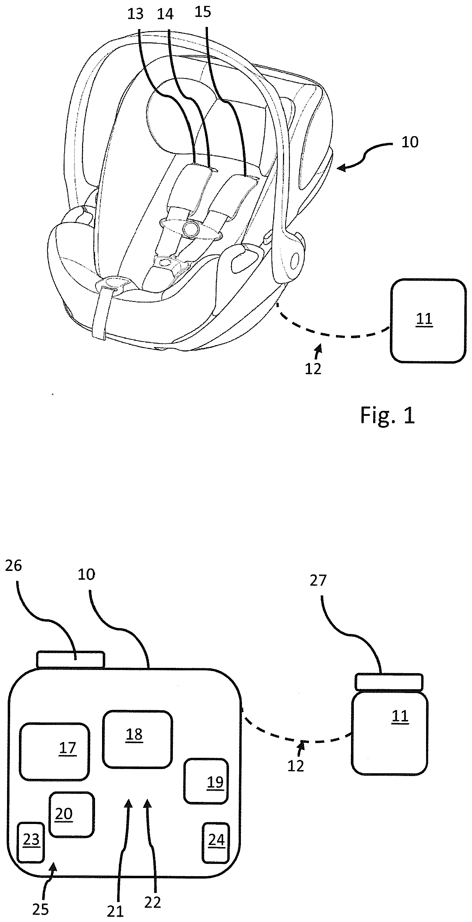

Fig. 1 zeigt einen Kindersitz 10 (in Schrägansicht) sowie eine zweite Überprüfungseinrichtung (z. B. in Form eines oder ausgebildet durch ein Smartphone) 11. Die zweite Überprüfungseinrichtung 11 kann mit dem Kindersitz 10 (bzw. Komponenten davon) drahtlos kommunizieren, über einen Pfad 12.

Der Kindersitz 10 weist ein Gurtsystem 13 auf. In Schultergurten 14, 15 ist (jeweils) ein Spannungssensor (z. B. umfassend ein elastisches Element, das seinen (elektrischen) Widerstand ändert, wenn es aufgrund einer anliegenden Spannungskraft gedehnt wird). In diesem Zusammenhang ist unter Spannung, soweit nichts anderes angegeben ist, immer eine mechanische Spannung zu verstehen.

Der (jeweilige) Spannungssensor misst vorzugsweise in zeitlichen Abständen von von 1 bis 1000 Millisekunden, z. B 100 Millisekunden, und ist mit einer Steuereinrichtung 17 (siehe Fig. 2) verbunden. Bestandteil der Steuereinrichtung 17 (oder dieser zugeordnet) ist eine Energieversorgung 18 (z. B. Batterie) und eine Signalsendeeinrichtung 19.

Die Steuereinrichtung 17 berechnet vorzugsweise alle 1 bis 100, z. B. alle 10, Sekunden einen Durchschnittswert und ggf. eine Standardabweichung der gemessenen Spannungswerte.

Der Spannungssensor 20 ist zusammen mit der Steuereinrichtung 17, der Energieversorgung 18 und der Signalsendeeinrichtung 19, Bestandteil einer ersten Überprüfungseinrichtung 21 und einer dritten Überprüfungseinrichtung 22.

Der Kindersitz 10 umfasst weiterhin, insbesondere zwei, (Widerstands-) Thermometer 23, 24. Jeweils ein Thermometer 23, 24 kann an einer Seite des Kindersitzes 10 angeordnet sein, vorzugsweise an einer jeweiligen seitlichen Außenfläche des Kindersitzes 10 (insbesondere in einem Bereich, der mit dem Kopf oder der Schulterpartie des Kindes in dem Kindersitz korrespondiert). Das jeweilige Thermometer 23, 24 misst die Temperatur der Umgebungsluft alle 1 bis 1000, z.B. alle 100, Millisekunden. Weiterhin sind die Thermometer 23, 24 mit der Steuereinrichtung 17 und der Signalsendeeinrichtung 19 verbunden. Die Steuereinrichtung 17 berechnet alle 1 bis 100, z. B. alle 10 Sekunden einen Durchschnitt der gemessenen Temperaturen. Das/die Thermometer, die Steuereinrichtung 17, die Energieversorgung 18 und die Signalsendeeinrichtung 19 sind Bestandteile einer vierten Überprüfungseinrichtung 25.

Die zweite Überprüfungseinrichtung 11 wird vorzugsweise durch ein mobiles Gerät (insbesondere Smartphone) ausgebildet (zumindest mit ausgebildet), wobei das Smartphone vorzugsweise eine entsprechende Software (App) aufweisen kann. Eine Zuordnung zwischen einem bestimmten Kindersitz und einer bestimmten zweiten Überprüfungseinrichtung 11 (Smartphone) kann durch ein grundsätzlich bekanntes Verfahren durchgeführt werden, wobei jeder Kindersitz einer oder mehreren zweiten Überprüfungseinrichtungen 11 zugeordnet werden kann, und/oder jede zweite Überprüfungseinrichtung (Smartphone) einem oder mehreren Kindersitzen zugeordnet sein kann.

Wenn der Kindersitz seinen Status hinsichtlich der Anwesenheit des Kindes ändert (von "frei" zu "besetzt"), kann ggf. eine (Bluetooth-) Verbindung zwischen dem Kindersitz und dem zugehörigen Smartphone aufgebaut werden. Wenn keine Verbindung aufgebaut werden kann (beispielsweise innerhalb einer vorbestimmten Zeit, von insbesondere 1 bis 50, z. B. 5, Sekunden) kann der Kindersitz einen (beispielsweise akustischen) Alarm aussenden, der ggf. nicht unterdrückt werden kann. Solange zumindest ein zugeordnetes Smartphone in einer (Bluetooth-) Verbindung mit dem Kindersitz ist, sendet der Kindersitz vorzugsweise keine Daten von den jeweiligen Sensoren zu der zweiten Überprüfungseinrichtung 11 (Smartphone). Wenn die Temperatur unter einen vorbestimmten unteren Grenzwert (von beispielsweise 10° oder weniger) fällt und/oder über einen vorbestimmten oberen Grenzwert (von beispielsweise mindestens 30° oder mindestens 35° oder mehr) ansteigt, insbesondere während die (Bluetooth-) Verbindung aufrechterhalten ist, kann der Kindersitz einen (insbesondere akustischen) Alarm aussenden, der ggf. (unmittelbar) am Kindersitz und/oder via zweiter Überprüfungseinrichtung 11 (Smartphone) einmal für 2 bis 20, insbesondere 10, Minuten unterdrückt werden kann.

Wenn die (Bluetooth-) Verbindung unterbrochen ist, während die Temperatur (immer noch) zu niedrig oder zu hoch ist, kann die Signalsendeeinrichtung 19 ggf. nach einer vorbestimmten Zeit (von beispielsweise mindestens 20 Sekunden oder mindestens 30 Sekunden oder mehr) die berechnete Durchschnittstemperatur zu der zweiten Überprüfungseinrichtung 11 (Smartphone) via Mobilverbindung (hier und im Folgenden: vorzugsweise Mobilfunknetzverbindung) senden, was die zweite Überprüfungseinrichtung 11 (Smartphone) dazu veranlassen kann, einen (akustischen und/oder visuellen und/oder vibratorischen) Alarm abzugeben. Dieser Alarm kann ggf. für eine bestimmte Zeitspanne (von beispielsweise 1 bis 5 Minuten, vorzugsweise 2 Minuten) unterdrückt werden.

Solange keine Anwesenheit eines Kindes im Kindersitz festgestellt wird, wird vorzugsweise keine (Bluetooth-) Verbindung zwischen dem Kindersitz und der zweiten Überprüfungseinrichtung 11 (Smartphone) aufgebaut und der Kindersitz 10 sendet dann insbesondere keine Daten zu der zweiten Prüfungseinrichtung 11 (Smartphone).

Wenn der Kindersitz besetzt ist (also ein Kind anwesend ist) und zwar durch ein schlafendes Kind (was insbesondere durch die Information festgestellt werden kann, die durch die Spannungssensoren bereitgestellt wird, insbesondere dadurch dass die durchschnittliche, gemessene Spannung einen ersten vorbestimmten Schwellwert überschreitet, während die Standardabweichung unter einem zweiten vorbestimmten Schwellwert bleibt), und die (Bluetooth-) Verbindung unterbrochen ist, während die Temperatur (immer noch) in einem zulässigen Bereich ist (z. B. innerhalb 10 bis 35° C), kann die Signalsendeeinrichtung 19 nach einer vorbestimmten Zeit (von beispielsweise 1 bis 5, insbesondere 2, Minuten) signalisieren, die letzte berechnete Information von (allen) beteiligten Sensoreinrichtungen zu der zweiten Überprüfungseinrichtung 11 (Smartphone) via Mobil-Verbindung zu senden, so dass die zweite Überprüfungseinrichtung 11 (Smartphone) einen akustischen und/oder visuellen und/oder vibratorischen Alarm abgibt. Dieser Alarm kann ggf. ein oder mehrmals (beispielsweise dreimal) für eine bestimmte Zeit (beispielsweise 5 bis 20, insbesondere 10, Minuten, als ggf. insgesamt 30 Minuten) unterdrückt werden.

Wenn der Kindersitz durch ein waches Kind besetzt ist und die (Bluetooth-) Verbindung unterbrochen ist, während die Temperatur immer noch in einem zulässigen Bereich ist, kann die Signalsendeeinrichtung 19 vom Kindersitz 10 nach einer vorbestimmten Zeit (z. B. 10 bis 100, insbesondere 30, Sekunden) die letzten berechneten Informationen von (allen) entsprechenden Sensoren des Kindersitzes 10 zu der zweiten Überprüfungseinrichtung 11 (Smartphone) via Mobil-Verbindung senden, so dass die zweite Überprüfungseinrichtung 11 (Smartphone) veranlasst wird, einen akustischen und/oder visuellen und/oder vibratorischen Alarm abzugeben. Dieser Alarm kann ggf. einmal (oder mehrmals) für eine bestimmte Zeit (beispielsweise 5 bis 20, insbesondere 10, Minuten) unterdrückt werden.

Wenn kein bzw. noch kein Signal gesendet wurde und die Temperatur außerhalb des zulässigen Bereichs fällt und/oder das Kind aufwacht, kann die Signalsendeeinrichtung 19 von dem Kindersitz 10 nach einer vorbestimmten Zeit (von beispielsweise 1 bis 100, insbesondere 30, Sekunden) die letzte berechnet Information von (allen) Sensoreinrichtungen des Kindersitzes 10 zu der zweiten Überprüfungseinrichtung 11 (Smartphone) via Mobil-Verbindung senden, so dass die zweite Überprüfungseinrichtung 11 (Smartphone) veranlasst wird, einen akustischen und/oder visuellen und/oder vibratorischen Alarm abzugeben. Dieser Alarm kann einmal (oder alternativ mehrmals) für eine vorbestimmte Zeit (von beispielsweise 1 bis 5, insbesondere 2, Minuten) unterdrückt werden (wenn er durch die Temperatur veranlasst wird) oder eine weitere, insbesondere abweichende, vorbestimmte Zeit von beispielsweise 5 bis 20, insbesondere 10, Minuten unterdrückt werden (wenn er durch ein Aufwachen des Kindes veranlasst wird). Vorzugsweise endet der Alarm nicht, wenn der Status des Kindes zurück zu "schlafend" geht und/oder wenn die Temperatur in einen zulässigen Bereich zurückkehrt.

Im Allgemeinen, wenn der Kindersitz 10 Daten über eine Mobil-Verbindung zu der zweiten Überprüfungseinrichtung 11 (Smartphone) sendet, kann er diese Daten vorzugsweise zu der letzten (Bluetooth-) verbundenen Überprüfungseinrichtung 11 (Smartphone) senden (oder einer Mehrzahl von zweiten Überprüfungseinrichtungen 11 bzw. Smartphones, wenn mehrere Überprüfungseinrichtungen 11 bzw. Smartphones ihre (Bluetooth-)Verbindung zu dem Kindersitz 10, insbesondere innerhalb einer bestimmte Zeitspanne von beispielsweise 2 bis 10, insbesondere 5, Minuten verloren haben).

Jeder Alarm, der von der zweiten Überprüfungseinrichtung 11 (Smartphone) ausgegeben wird, endet vorzugsweise automatisch sobald eine (Bluetooth-) Verbindung zwischen dem Kindersitz und einer zugeordneten zweiten Überprüfungseinrichtung 11 (Smartphone) wiederhergestellt wird. Abgesehen davon ist es möglich, das gesamte System unmittelbar am Sitz auszuschalten (allerdings vorzugsweise nicht über die zweite Überprüfungseinrichtung 11 bzw. das Smartphone), was alle Alarme beendet (auch die, die grundsätzlich, zumindest über die zweite Überprüfungseinrichtung 11 bzw. das Smartphone, nicht unterdrückt werden können). Vor einem Ausschalten erfolgt dann vorzugsweise eine Nachricht an alle zugeordneten zweiten Überprüfungseinrichtungen 11 (Smartphones) via Mobil-Verbindung.

Das Unterdrücken eines Alarms (wenn nicht ausgeschlossen) kann durch die zweite Überprüfungseinrichtung 11 (Smartphone) oder unmittelbar am Kindersitz erfolgen. Wenn es unmittelbar am Kindersitz erfolgt wird vorzugsweise eine entsprechende Nachricht an alle zugeordneten zweiten Überprüfungseinrichtungen 11 (Smartphones) via Mobil-Verbindung gesendet.

Die Steuereinrichtung 17 kann (teilweise oder gänzlich) auch durch die zweite Überprüfungseinrichtung 11 (bzw. Smartphone) ausgebildet werden.

Der Kindersitz weist vorzugsweise eine Anzeigeeinrichtung 26 (insbesondere Display) auf. Alternativ oder zusätzlich kann auch die zweite Überprüfungseinrichtung eine Anzeigeeinrichtung 27 (insbesondere Display) aufweisen.

Die Steuereinrichtung kann, wenn sie mit den folgenden Situationen konfrontiert wird, beispielsweise wie folgt reagieren:

| Kindersitz besetzt (KiKS)? | Betreuende Person anwesend (Ba)? | Kind wach (Kw)? | Temperatur OK (TiaB)? | Alarm |
|---|---|---|---|---|
| Nein | Beliebig | Beliebig | Beliebig | Kein Alarm |
| Ja | Beliebig | Beliebig | Nein | Alarm |
| Ja | Ja | Beliebig | Ja | Kein Alarm |
| Ja | Nein | Ja | Ja | Alarm |
| Ja | Nein | Nein | Ja | Kein Alarm |

Die obigen Regeln können modifiziert und/oder vervollständigt werden. In bevorzugten Ausführungsformen können eine oder mehrere der folgenden Regeln (durch die Steuereinrichtung) eingehalten werden:
- Ein Alarm wird niemals veranlasst, wenn kein Kind in dem Kindersitz ist.
- Ein Alarm wird immer veranlasst, wenn ein Kind in dem Kindersitz ist und die Temperatur zu hoch oder zu niedrig ist.
- Ein Alarm wird immer veranlasst, wenn ein Kind in dem Kindersitz ist, das Kind wach ist und keine betreuende Person anwesend ist.
- Ein Alarm wird nach einer vorbestimmten Zeitspanne veranlasst, wenn ein Kind in dem Kindersitz ist und das Kind schläft und keine betreuende Person anwesend ist.
- Ein Alarm kann nicht (oder nur für eine, insbesondere vergleichsweise kurze, Zeit) unterdrückt werden, wenn er aufgrund eines Temperatur-Status veranlasst wird.
- Ein Alarm kann für eine vorbestimmte Zeit nicht unterdrückt werden, wenn er durch die Abwesenheit einer betreuenden Person und/oder aufgrund eines Aufwachens des Kindes veranlasst wird.
- Der Status des Kindes kann sich nur dann von "wach" zu "schlafend" ändern, wenn eine betreuende Person anwesend ist.

### Bezugszeichen

- 10: Kindersitz
- 11: zweite Überprüfungseinrichtung
- 12: Pfad
- 13: Gurtsystem
- 14: Schultergurt
- 15: Schultergurt
- 17: Steuereinrichtung
- 18: Energieversorgung
- 19: Signalsendeeinrichtung
- 20: Spannungssensor
- 21: erste Überprüfungseinrichtung
- 22: dritte Überprüfungseinrichtung
- 23: Thermometer
- 24: Thermometer
- 25: vierte Überprüfungseinrichtung
- 26: Anzeigeeinrichtung
- 27: Anzeigeeinrichtung

## Patentansprüche

1. Kindersitzüberprüfungssystem für einen Kindersitz (10) eines Fahrzeugs, insbesondere Kraftfahrzeuges, umfassend zumindest einen Kindersitz (10) sowie eine erste Überprüfungseinrichtung (21) zum Überprüfen, ob sich ein Kind in dem Kindersitz befindet, und eine zweite Überprüfungseinrichtung (11) zum Überprüfen, ob eine das Kind betreuende Person anwesend ist, wobei weiterhin eine dritte (22) Überprüfungseinrichtung vorgesehen ist, wobei die dritte Überprüfungseinrichtung zur Überprüfung eines Wachheitsstatus des Kindes ausgebildet ist, wobei mindestens eine Steuereinrichtung (17) vorgesehen ist, die konfiguriert ist, in Abhängigkeit einer Überprüfung durch die erste (21), zweite (11) und dritte (22) Überprüfungseinrichtung, mindestens ein Ergebnis aus einem Vergleich der Überprüfungen der ersten, zweiten und dritten Überprüfungseinrichtungen auszugeben, wobei die erste (21), zweite (11) und dritte (22) Überprüfungseinrichtung eine jeweilige Ausgabeeinrichtung umfassen, zur Ausgabe eines Ergebnisses der Überprüfungen an die mindestens eine Steuereinrichtung (17), wobei die Steuereinrichtung (17) weiter dazu konfiguriert ist das jeweilige Ergebnis an eine, vorzugsweise akustische und/oder optische, Anzeigeeinrichtung auszugeben.

2. Kindersitzüberprüfungssystem nach Anspruch 1, umfassend eine vierte Überprüfungseinrichtung (25) zur Überprüfung einer Kindersitzumgebung, insbesondere einer Kindersitzumgebungstemperatur.

3. Kindersitzüberprüfungssystem nach Anspruch 1 oder 2, wobei die zweite Überprüfungseinrichtung in einem Betriebszustand des Kindersitzüberprüfungssystems zumindest teilweise tragbar und/oder unabhängig von einem Ein-/Auszustand des Fahrzeuges arbeitend ausgebildet ist.

4. Kindersitzüberprüfungssystem nach Anspruch 2,
**dadurch gekennzeichnet, dass**
die mindestens eine Steuereinrichtung (17) konfiguriert ist, in Abhängigkeit einer Überprüfung durch die vierte (25) Überprüfungseinrichtung, mindestens ein Ergebnis aus der jeweiligen Überprüfung und/oder mindestens ein Ergebnis aus einem Vergleich von mehreren Überprüfungen auszugeben, insbesondere an die, vorzugsweise akustische und/oder optische, Anzeigeeinrichtung.

5. Kindersitzüberprüfungssystem nach Anspruch 2 und optional zusätzlich nach Anspruch 3, oder 4,
**dadurch gekennzeichnet, dass**
die vierte (25) Überprüfungseinrichtung eine Ausgabeeinrichtung umfasst, zur Ausgabe eines Ergebnisses der Überprüfungen, insbesondere an die mindestens eine Steuereinrichtung.

6. Kindersitzüberprüfungssystem nach einem der vorhergehenden Ansprüche,
**dadurch gekennzeichnet, dass**
mindestens eine, insbesondere akustische und/oder optische, vorzugsweise eine Baueinheit mit der zweiten Überprüfungseinrichtung oder zumindest Teilen davon ausbildende, Anzeigeeinrichtung, vorgesehen ist, die konfiguriert ist, in Abhängigkeit einer Überprüfung durch die erste, zweite, dritte und/oder vierte Überprüfungseinrichtung, mindestens ein Ergebnis der jeweiligen Überprüfung oder mindestens ein Ergebnis aus einem Vergleich von mehreren Überprüfungen anzuzeigen, vorzugsweise sämtliche Ergebnisse aus den Überprüfungen der Überprüfungseinrichtungen anzuzeigen und/oder ein Alarmsignal auszugeben, falls bzw. sobald ein unerwünschter Zustand im Hinblick auf das Kind und/oder die Anwesenheit der betreuenden Person festgestellt wird.

7. Kindersitzüberprüfungssystem nach einem der vorhergehenden Ansprüche,
**dadurch gekennzeichnet, dass**
die erste (21), dritte (22) und/oder vierte (25) Überprüfungseinrichtungen zumindest teilweise an dem oder in dem Kindersitz (10) angeordnet sind, wobei die vierte Überprüfungseinrichtung (25) vorzugsweise zumindest teilweise an einer Außenseite des Kindersitzes angeordnet ist.

8. Kindersitzüberprüfungssystem nach einem der vorhergehenden Ansprüche,
**dadurch gekennzeichnet, dass**
die erste Überprüfungseinrichtung (21) mindestens einen Rückhalteeinrichtungssensor umfasst, der konfiguriert ist, einen Status mindestens einer Rückhalteeinrichtung, wie beispielsweise eines Haltegurtes und/oder eines Fangkörpers, festzustellen und/oder mindestens einen Gewichtssensor und/oder mindestens eine optische Einrichtung, wie z.B. eine Kamera, umfasst.

9. Kindersitzüberprüfungssystem nach einem der vorhergehenden Ansprüche,
**dadurch gekennzeichnet, dass**
die zweite Überprüfungseinrichtung (11) mindestens einen fahrzeugeigenen Sensor, wie beispielsweise einen Gewichtssensor und/oder einen Zündsensor, umfasst und/oder
ein mobiles Gerät, wie beispielsweise ein Mobiltelefon, insbesondere Smartphone, und/oder eine Uhr, insbesondere Smartwatch, und/oder einen Activity Tracker und/oder eine Seh- und/oder Hörhilfe und/oder einen, insbesondere elektrischen bzw. elektronischen, Zündschlüssel und/oder ein sonstiges mobiles Gerät, umfassend einen Transmitter und/oder Receiver und/oder Transponder, umfasst und/oder eine Abstandsbestimmungseinrichtung umfasst, die konfiguriert ist, einen Abstand zwischen dem Kindersitz (10) und einem/dem mobilen Gerät, wie beispielsweise einem Mobiltelefon, insbesondere Smartphone, und/oder einer Uhr, insbesondere Smartwatch, und/oder einem Activity Tracker und/oder einer Seh- und/oder Hörhilfe und/oder einem, insbesondere elektrischen bzw. elektronischen, Zündschlüssel und/oder einem sonstigen mobilen Gerät, umfassend einen Transmitter und/oder Receiver und/oder Transponder, zu bestimmen.

10. Kindersitzüberprüfungssystem nach einem der vorhergehenden Ansprüche,
**dadurch gekennzeichnet, dass**
die dritte Überprüfungseinrichtung (22) mindestens einen Bewegungssensor und/oder eine Kamera und/oder einen Geräuschsensor und/oder mindestens einen Gewichtssensor und/oder mindestens einen Sensor zur Bestimmung eines physischen Zustandes des Kindes, wie z. B. ein Pulssensor und/oder ein Atmungssensor, umfasst.

11. Kindersitzüberprüfungssystem nach einem der vorhergehenden Ansprüche,
**dadurch gekennzeichnet, dass**
die erste (21), zweite (11), dritte (22) und/oder vierte (25) Überprüfungseinrichtung mehrere Sensoren und/oder ein Sensor-Array umfasst/umfassen.

12. Kindersitzüberprüfungssystem nach einem der vorhergehenden Ansprüche,
**dadurch gekennzeichnet, dass**
die erste (21), zweite (11), dritte (22) und/oder vierte (25) Überprüfungseinrichtung und/oder Steuereinrichtung und/oder Anzeigeeinrichtung zur drahtlosen Kommunikation, insbesondere über Funk und/oder Bluetooth und/oder Infrarot, und/oder zur internetbasierten Kommunikation ausgebildet sind.

13. Kindersitzüberprüfungssystem nach einem der vorhergehenden Ansprüche,
**dadurch gekennzeichnet, dass**
der Kindersitz und die zweite Überprüfungseinrichtung zur drahtlosen Kommunikation über eine erste drahtlose Kommunikationsverbindung, z. B. über Bluetooth, ausgebildet sind, wobei eine Messung, z. B. Abstandsmessung, auf Grundlage der ersten Kommunikationsverbindung über die erste Kommunikationsverbindung oder eine zweite drahtlose Kommunikationsverbindung, z. B. eine Mobilfunkverbindung, kommuniziert wird und/oder, wobei bei einer Unterbrechung der ersten drahtlosen Kommunikationsverbindung eine Information, insbesondere ein entsprechender Alarm generiert wird, vorzugsweise automatisch oder durch Kommunizieren mittels der zweiten drahtlosen Kommunikationsverbindung.

14. Kindersitzüberprüfungsverfahren für einen Kindersitz eines Fahrzeuges, insbesondere Kraftfahrzeuges, unter Verwendung eines Kindersitzüberprüfungssystems nach einem der vorhergehenden Ansprüche 1 bis 13,
umfassend:
a) Überprüfen, ob sich ein Kind in einem Kindersitz (10) befindet;
b) Überprüfen, ob eine das Kind betreuende Person anwesend ist; und
c) Überprüfen eines Wachheitsstatus des Kindes, wobei eine Steuereinrichtung (17) in Abhängigkeit einer Überprüfung durch die erste (21), zweite (11) und dritte (22) Überprüfungseinrichtung, mindestens ein Ergebnis aus einem Vergleich der Überprüfungen der ersten, zweiten und dritten Überprüfungseinrichtungen ausgibt.

15. Kindersitzüberprüfungsverfahren nach Anspruch 14, wobei im Schritt b)
zumindest teilweise eine Überprüfungseinrichtung (11) zum Einsatz kommt, die - auch während des Überprüfens - tragbar und/oder unabhängig vom Betriebszustand des Fahrzeuges arbeitend ausgebildet ist.

16. Kindersitzüberprüfungsverfahren nach Anspruch 14 oder 15, umfassend:
Überprüfen einer Kindersitzumgebung, insbesondere einer Kindersitzumgebungstemperatur.

17. Kindersitzüberprüfungsverfahren nach einem der vorhergehenden Ansprüche 14 bis 16,
**dadurch gekennzeichnet, dass** eine, vorzugsweise optische und/oder akustische, Anzeige, insbesondere ein, vorzugsweise optischer und/oder akustischer, Alarm, erfolgt, insbesondere ohne dass weitere Bedingungen vorliegen müssen, wenn:
KiKS = ja, (Ba = beliebig,) (Kw = beliebig,) TiaB = nein; und/oder
KiKS = ja, Ba = nein, ggf. Kw = ja, (TiaB = beliebig); und/oder
dass eine, vorzugsweise optische und/oder akustische, Anzeige, insbesondere ein, vorzugsweise optischer und/oder akustischer, Alarm, vorzugsweise nicht erfolgt oder erst erfolgt, wenn eine vorbestimmte Zeit verstrichen ist, und wenn:
KiKS = ja, Ba = nein, ggf. Kw = nein, (TiaB = beliebig, insbesondere ja); und/oder
dass eine, vorzugsweise optische und/oder akustische, Anzeige, insbesondere ein, vorzugsweise optischer und/oder akustischer, Alarm, nicht erfolgt, wenn:
KiKS = nein, (Ba = beliebig,) (Kw = beliebig,) (TiaB = beliebig); und/oder
KiKS = ja, Ba = ja, (Kw = beliebig,) ggf. TiaB = ja; und/oder
die Anzeige bzw. der Alarm von der betreuenden Person unterdrückt wird, was vorzugsweise zumindest für eine Zeitspanne t1 möglich ist, insbesondere wenn Ba = nein und/oder Kw = ja bzw. wenn das Kind aufwacht und/oder was nicht oder nur für eine vorübergehende Zeitspanne t2, vorzugsweise kleiner t1, möglich ist, wenn die Temperatur nicht in einem akzeptablen Bereich liegt,
wobei KiKS = Kind in Kindersitz, Ba = Betreuer anwesend, Kw = Kind wach, Temperatur in akzeptablen Bereich = TiaB.

18. Kindersitzüberprüfungsverfahren nach einem der vorhergehenden Ansprüche 14 bis 17,
**dadurch gekennzeichnet, dass**
ein Statuswechsel von Kind = wach zu Kind = schlafend nur erfolgt, wenn die betreuende Person anwesend ist.

## Claims

1. Child safety seat checking system for a child safety seat (10) of a vehicle, in particular a motor vehicle, comprising at least one child safety seat (10) and a first checking device (21) for checking whether a child is in the child safety seat, and a second checking device (11) for checking whether a person caring for the child is present, wherein a third (22) checking device is also provided, wherein the third checking device is designed to check a wakefulness status of the child, wherein at least one control device (17) is provided which is configured to output, depending on a check by the first (21), second (11) and third (22) checking device, at least one result from a comparison of the checks of the first, second and third checking devices, wherein the first (21), second (11) and third (22) checking devices comprise a respective output device for outputting a result of the checks to the at least one control device (17), wherein the control device (17) is further configured to output the respective result to a, preferably acoustic and/or optical, display device.

2. Child safety seat checking system according to claim 1, comprising a fourth checking device (25) for checking a child safety seat environment, in particular a child safety seat ambient temperature.

3. Child safety seat checking system according to claim 1 or 2, wherein the second checking device is designed to be at least partially portable and/or to operate independently of an on/off state of the vehicle in an operating state of the child safety seat checking system.

4. Child safety seat checking system according to claim 2,
**characterized in that**
the at least one control device (17) is configured, depending on a check by the fourth (25) checking device, to output at least one result from the respective check and/or at least one result from a comparison of a plurality of checks, in particular to the, preferably acoustic and/or optical, display device.

5. Child safety seat checking system according to claim 2 and optionally additionally according to claim 3, or 4,
**characterized in that**
the fourth (25) checking device comprises an output device for outputting a result of the checks, in particular to the at least one control device.

6. Child safety seat checking system according to one of the preceding claims,
**characterized in that**
at least one, in particular acoustic and/or optical display device, preferably forming a structural unit with the second checking device or at least parts thereof, is provided, which is configured, depending on a check by the first, second, third and/or fourth checking device, to display at least one result of the respective check or at least one result from a comparison of a plurality of checks, preferably to display all results from the checks of the checking devices and/or to output an alarm signal if or as soon as an undesirable condition is detected with regard to the child and/or the presence of the caregiver.

7. Child safety seat checking system according to one of the preceding claims,
**characterized in that**
the first (21), third (22) and/or fourth (25) checking devices are arranged at least partially on or in the child safety seat (10), wherein the fourth checking device (25) is preferably arranged at least partially on an outside of the child safety seat.

8. Child safety seat checking system according to one of the preceding claims,
**characterized in that**
the first checking device (21) comprises at least one restraint device sensor which is configured to detect a status of at least one restraint device, such as a tether and/or a catch body, and/or comprises at least one weight sensor and/or at least one optical device, such as a camera.

9. Child safety seat checking system according to one of the preceding claims,
**characterized in that**
the second checking device (11) comprises at least one in-vehicle sensor, such as a weight sensor and/or an ignition sensor, and/or a mobile device, such as a mobile telephone, in particular a smartphone, and/or a watch, in particular a smartwatch, and/or an activity tracker and/or a visual and/or hearing aid and/or an ignition key, in particular an electric or electronic ignition key, and/or any other mobile device, comprising a transmitter and/or receiver and/or transponder, and/or comprises a distance determination device which is configured to determine a distance between the child safety seat (10) and a/the mobile device, such as a mobile telephone, in particular a smartphone, and/or a watch, in particular a smartwatch, and/or an activity tracker and/or a visual and/or hearing aid and/or an ignition key, in particular an electric or electronic ignition key, and/or other mobile device, comprising a transmitter and/or receiver and/or transponder.

10. Child safety seat checking system according to one of the preceding claims, **characterized in that**
the third checking device (22) comprises at least one motion sensor and/or a camera and/or a sound sensor and/or at least one weight sensor and/or at least one sensor for determining a physical state of the child, such as a pulse sensor and/or a respiration sensor.

11. Child safety seat checking system according to one of the preceding claims, **characterized in that**
the first (21), second (11), third (22) and/or fourth (25) checking device comprises a plurality of sensors and/or a sensor array.

12. Child safety seat checking system according to one of the preceding claims, **characterized in that**
the first (21), second (11), third (22) and/or fourth (25) checking device and/or control device and/or display device are designed for wireless communication, in particular via radio and/or Bluetooth and/or infrared, and/or for internet-based communication.

13. Child safety seat checking system according to one of the preceding claims, **characterized in that**
the child safety seat and the second checking device are designed for wireless communication via a first wireless communication link, e.g. via Bluetooth, wherein a measurement, e.g. distance measurement, is performed on the basis of the first communication link via the first communication link or a second wireless communication link, e.g. via a mobile network connection, and/or, wherein, in the event of an interruption of the first wireless communication link, information, in particular a corresponding alarm, is generated, preferably automatically or by communicating by means of the second wireless communication link.

14. Child safety seat checking method for a child safety seat of a vehicle, in particular motor vehicle, using a child safety seat checking system according to one of the preceding claims 1 to 13, comprising:
a) checking whether a child is in a child safety seat (10);
b) checking whether a person caring for the child is present; and
c) checking a wakefulness status of the child, wherein a control device (17) outputs, depending on a check by the first (21), second (11) and third (22) checking device, at least one result from a comparison of the checks of the first, second and third checking devices.

15. Child safety seat checking method according to claim 14, wherein in step b) at least partly a checking device (11) is used which - also during the checking - is designed to be portable and/or to operate independently of the operating state of the vehicle.

16. Child safety seat checking method according to claim 14 or 15, comprising: checking a child safety seat environment, in particular a child safety seat ambient temperature.

17. Child safety seat checking method according to one of the preceding claims 14 to 16, **characterized in that**
a, preferably optical and/or acoustic, display, in particular a, preferably optical and/or acoustic, alarm, takes place, in particular without further conditions having to be present, if:
CiCSS = yes, (Cp = any,) (Ca = any,) TwaR = no; and/or CiCSS = yes, Cp = no,
optionally Ca = yes, (TwaR = any); and/or
**in that** a, preferably visual and/or acoustic, display, in particular a, preferably visual and/or acoustic, alarm, preferably does not occur or only occurs when a predetermined time has elapsed, and if:
CiCSS = yes, Cp = no, optionally Ca = no, (TwaR = any, in particular yes); and/or
**in that** a, preferably visual and/or acoustic, display, in particular a, preferably visual and/or acoustic, alarm, does not occur if:
CiCSS = no, (Cp = any,) (Ca = any,) (TwaR = any); and/or
CiCSS = yes, Cp = yes, (Ca = any,) optionally TwaR = yes; and/or
the display or alarm is suppressed by the caregiver, which is preferably possible for at least a period of time t1, in particular if Cp = no and/or Ca = yes or if the child wakes up and/or which is not possible or only possible for a temporary period of time t2, preferably less than t1, if the temperature is not within an acceptable range,
wherein CiCSS = child in child safety seat, Cp = caregiver present, Ca = child awake, TwaR = temperature within acceptable range.

18. Child safety seat checking method according to one of the preceding claims 14 to 17, **characterized in that**
a status change from child = awake to child = asleep only takes place when the caregiver is present.

## Revendications

1. Système de vérification de siège enfant pour un siège enfant (10) d'un véhicule, en particulier d'un véhicule à moteur, comprenant au moins un siège enfant (10) et un premier dispositif de vérification (21) destiné à vérifier s'il y a un enfant dans le siège enfant, ainsi qu'une deuxième dispositif de vérification (11) destiné à vérifier si une personne qui s'occupe de l'enfant est présente, dans lequel est prévu en outre un troisième dispositif de vérification (22), le troisième dispositif de vérification étant conçu pour vérifier l'état de veille de l'enfant, dans lequel est prévu un dispositif de commande (17) qui est configuré pour transmettre en sortie, en fonction d'une vérification par le premier dispositif de vérification (21), le deuxième (11) et le troisième (22), au moins un résultat d'une comparaison des vérifications des premier, deuxième et troisième dispositifs de vérification, le premier dispositif de vérification (21), le deuxième (11) et le troisième (22) comprenant chacun un dispositif de sortie pour transmettre en sortie un résultat des vérifications à l'au moins un dispositif de commande (17), lequel dispositif de commande (17) est en outre configuré pour transmettre le résultat en question en sortie à un dispositif indicateur, de préférence sonore et/ou visuel.

2. Système de vérification de siège enfant selon la revendication 1, comprenant un quatrième dispositif de vérification (25) pour la vérification d'un environnement du siège enfant, en particulier d'une température dans l'environnement du siège enfant.

3. Système de vérification de siège enfant selon la revendication 1 ou 2, dans lequel le deuxième dispositif de vérification est au moins en partie portatif dans un état de fonctionnement du système de vérification de siège enfant et/ou conçu pour fonctionner indépendamment d'un état de marche ou d'arrêt du véhicule.

4. Système de vérification de siège enfant selon la revendication 2, **caractérisé en ce que** l'au moins un dispositif de commande (17) est configuré pour transmettre en sortie, en fonction d'une vérification par le quatrième (25) dispositif de vérification, au moins un résultat de la vérification correspondante et/ou au moins un résultat d'une comparaison de plusieurs vérifications, en particulier au dispositif indicateur, de préférence sonore et/ou visuel.

5. Système de vérification de siège enfant selon la revendication 2 et, facultativement, selon la revendication 3 ou 4, **caractérisé en ce que** le quatrième (25) dispositif de vérification comprend un dispositif de sortie pour transmettre en sortie un résultat des vérifications, en particulier à l'au moins un dispositif de commande.

6. Système de vérification de siège enfant selon l'une des revendications précédentes, **caractérisé en ce qu'**est prévu au moins un dispositif indicateur, en particulier sonore et/ou visuel, formant de préférence une unité de construction avec le deuxième dispositif de vérification ou formant au moins des parties de celui-ci, qui est configuré pour indiquer, en fonction d'une vérification par le premier, deuxième, troisième et/ou quatrième dispositif de vérification, au moins un résultat de la vérification en question ou au moins un résultat d'une comparaison de plusieurs vérifications, de préférence tous les résultats des vérifications des dispositifs de vérification, et/ou émettre un signal d'alarme si et dès qu'un état indésirable pour l'enfant et/ou la présence de la personne qui s'occupe de lui est constaté.

7. Système de vérification de siège enfant selon l'une des revendications précédentes, **caractérisé en ce que** les premier (21), troisième (22) et/ou quatrième (25) dispositifs de vérification sont au moins en partie disposés dans ou sur le siège enfant (10), le quatrième dispositif de vérification (25) étant de préférence disposé au moins en partie sur un côté extérieur du siège enfant.

8. Système de vérification de siège enfant selon l'une des revendications précédentes, **caractérisé en ce que** le premier dispositif de vérification (21) comprend au moins un capteur de dispositif de retenue configuré pour déterminer un état d'au moins un dispositif de retenue, par exemple d'une ceinture de sécurité ou d'un bouclier d'impact et/ou d'au moins un capteur de poids et/ou d'au moins un dispositif optique, par exemple d'une caméra.

9. Système de vérification de siège enfant selon l'une des revendications précédentes, **caractérisé en ce que** le deuxième dispositif de vérification (11) comprend au moins un capteur propre au véhicule, par exemple un capteur de poids et/ou un détecteur d'allumage, et/ou un appareil mobile, par exemple un téléphone mobile, en particulier un smartphone, et/ou une montre, en particulier une montre connectée, et/ou un moniteur d'activité physique et/ou une aide visuelle et/ou auditive et/ou une clé d'allumage, en particulier électrique ou électronique, et/ou un autre appareil mobile, comprenant un émetteur et/ou un récepteur et/ou un transpondeur, et/ou comprend un dispositif de détermination de la distance qui est configuré pour déterminer une distance entre le siège enfant et un appareil ou l'appareil mobile, par exemple un smartphone, et/ou une montre, en particulier une montre connectée, et/ou un traceur d'activité et/ou une aide visuelle et/ou auditive et/ou une clé d'allumage, en particulier électrique ou électronique, et/ou un autre appareil mobile, comprenant un émetteur et/ou un récepteur et/ou un transpondeur.

10. Système de vérification de siège enfant selon l'une des revendications précédentes, **caractérisé en ce que** le troisième dispositif de vérification (22) comprend au moins un détecteur de mouvement et/ou une caméra et/ou un détecteur de bruit et/ou au moins un capteur de poids et/ou au moins un capteur destiné à déterminer un état physique de l'enfant, par exemple un capteur de pouls et/ou un capteur de respiration.

11. Système de vérification de siège enfant selon l'une des revendications précédentes, **caractérisé en ce que** le premier dispositif de vérification (21), le deuxième (11), le troisième (22) et/ou le quatrième (25) comporte(nt) plusieurs capteurs et/ou une matrice de capteurs.

12. Système de vérification de siège enfant selon l'une des revendications précédentes, **caractérisé en ce que** le premier dispositif de vérification (21), le deuxième (11), le troisième (22) et/ou le quatrième (25) et/ou le dispositif de commande et/ou le dispositif indicateur sont conçus en vue d'une communication sans fil, en particulier par radio et/ou Bluetooth et/ou infrarouge, et/ou d'une communication par Internet.

13. Système de vérification de siège enfant selon l'une des revendications précédentes, **caractérisé en ce que** le siège enfant et le deuxième dispositif de vérification sont conçus en vue d'une communication sans fil par une première liaison de communication sans fil, par exemple par Bluetooth, une mesure, par exemple une détermination de distance, étant communiquée sur la base de la première liaison de communication sur la première liaison de communication ou une deuxième liaison de communication sans fil, par exemple une liaison par radiotéléphonie et/ou une information, en particulier une alarme correspondante, étant générée en cas d'interruption de la première liaison de communication sans fil, de préférence automatiquement ou par une communication au moyen de la deuxième liaison de communication sans fil.

14. Procédé de vérification de siège enfant pour un siège enfant d'un véhicule, en particulier d'un véhicule à moteur, utilisant un système de vérification de siège enfant selon l'une des revendications 1 à 13, comprenant :
a) la vérification de la présence d'un enfant dans un siège enfant (10) ;
b) la vérification de la présence d'une personne qui s'occupe de l'enfant et
c) la vérification d'un état de veille de l'enfant,
dans lequel un dispositif de commande (17) transmet en sortie, en fonction d'une vérification par le premier dispositif de vérification (21), le deuxième (11) et le troisième (22), au moins un résultat d'une comparaison des premier, deuxième et troisième dispositifs de vérification.

15. Procédé de vérification de siège enfant selon la revendication 14, dans lequel est au moins en partie utilisé dans l'étape b) un dispositif de vérification (11) qui est conçu pour être portable et/ou pour pouvoir fonctionner indépendamment de l'état de fonctionnement du véhicule, même pendant la vérification.

16. Procédé de vérification de siège enfant selon la revendication 14 ou 15, comprenant la vérification d'un environnement du siège enfant, en particulier d'une température dans l'environnement du siège enfant.

17. Procédé de vérification de siège enfant selon l'une des revendications 14 à 16, **caractérisé en ce qu'**un indicateur, de préférence visuel et/ou sonore, en particulier une alarme, de préférence visuelle et/ou sonore, est activé, en particulier sans nécessiter d'autres conditions, si :
ES = oui, (PP = indifférent), (EE = indifférent), TPA = non et/ou
ES = oui, PP = non, éventuellement EE = oui, (TPA = indifférent)
et/ou **en ce qu'**un indicateur, de préférence visuel et/ou sonore, en particulier une alarme, de préférence visuelle et/ou sonore, n'est de préférence pas activé ou n'est activé qu'après un temps prédéterminé et si :
ES = oui, PP = non, éventuellement EE = non, (TPA = indifférent, en particulier oui) et/ou
**en ce qu'**un indicateur, de préférence visuel et/ou sonore, en particulier une alarme, de préférence visuelle et/ou sonore, n'est pas activé si :
ES = non, (PP = indifférent), (EE = indifférent), (TPA = indifférent) et/ou
ES = oui, PP = oui, (EE = indifférent,) éventuellement TPA = oui
et/ou l'indicateur ou l'alarme est inhibé par la personne qui s'occupe de l'enfant, ce qui est possible de préférence au moins pendant un laps de temps t1, en particulier si PP = non et/ou TPA = oui ou si l'enfant se réveille, et/ou n'est pas possible, ou seulement provisoirement pendant un laps de temps t2, de préférence plus court que t1, si la température ne se situe pas dans une plage acceptable,
où ES = enfant dans le siège auto, PP = présence de la personne qui s'occupe de l'enfant, EE = enfant éveillé, TPA = température dans la plage acceptable.

18. Procédé de vérification de siège enfant selon l'une des revendications précédentes 14 à 17, **caractérisé en ce qu'**un changement d'état d'***enfant** = **éveillé*** à ***enfant** = **endormi*** n'a lieu que si la personne qui s'occupe de l'enfant est présente.
